# EUROPEAN PATENT APPLICATION

(11) **EP 1 721 909 A1**
(43) Date of publication of application: **15.11.2006**
(21) Application number: 05010172.4
(22) Date of filing: 10.05.2005
(51) Int. Cl.: C07K 14/51, C12N 15/12, C07K 16/22, A61K 48/00, A61K 38/18, A61K 39/00

(54) **Growth factor mutants with altered biological attributes**

(71) Applicant: BIOPHARM GESELLSCHAFT ZUR BIOTECHNOLOGISCHEN ENTWICKLUNG VON PHARMAKA mbH, 69115 Heidelberg (DE); Charité - Universitätsmedizin Berlin, 10117 Berlin (DE); Freie Universität Berlin, 14195 Berlin (DE)
(72) Inventor: Mundlos, Stefan, Prof., 14532 Kleinmachnow (DE); Knaus, Petra, Prof., 14195 Berlin (DE); Pohl, Jens, Dr., 76707 Hambrücken (DE); Kruse, Michael, Dr., 55130 Mainz (DE)
(74) Representative: Böhm, Brigitte

(57) **Abstract**

The invention relates to novel recombinant growth factor mutants with altered biological attributes which are derived from proteins of the TGF-β superfamily of signalling molecules and exhibit novel antagonistic properties. They are particularly suitable for the determination of cell fates, the growth stimulation and differentiation of various cells including stem cells and the regeneration of organs. They are also useful for the supportive therapy of muscular diseases and treatment of disorders characterized by the excessive action of TGF-β superfamily members.

## Description

The invention relates to novel recombinant growth factor mutants with altered biological attributes. The growth factor mutants provided herein are derived from proteins of the TGF-ß superfamily of signalling molecules and exhibit novel antagonistic properties. They are particularly suitable for the determination of cell fates, the growth stimulation and differentiation of various cells including stem cells and the regeneration of organs. Further indications are supportive therapy of muscular diseases and treatment of disorders characterized by the excessive action of TGF-ß superfamily members. The invention also relates to nucleic acid molecules coding for said recombinant protein mutants, expression vectors and host cells containing the nucleic acid molecules, antibodies directed against said growth factor mutants, pharmaceutical compositions and methods for producing the growth factor mutants.

The transforming growth factor-beta (TGF-beta) superfamily of proteins comprises more than 35 members including TGF-betas, bone morphogenetic proteins (BMPs), activins, inhibins and growth/differentiation factors (GDFs). TGF-beta superfamily proteins promote cell proliferation and differentiation as well as tissue formation/regeneration and are relevant for a wide range of medical treatment methods and applications. These dimeric molecules act through specific receptor complexes that are composed of type I and type II serine/threonine receptor kinases. The receptor kinases subsequently activate Smad proteins, which then propagate the signals into the nucleus to regulate target gene expression. Smad independent signalling pathways are also initiated by these receptors and result in induction of the MAP Kinase pathway. Smads are a unique family of signal transduction molecules that can transmit signals directly from the cell surface receptors to the nucleus, where they regulate transcription by interacting with DNA binding partners as well as transcriptional coactivators and corepressors.

The members of this protein family are initially synthesized as large heterogeneous precursor proteins which subsequently undergo proteolytic cleavage at a cluster of basic residues approximately 110-140 amino acids from the C-terminus, thus releasing the C-terminal mature protein parts from the N-terminal prodomain. All mature polypeptides are structurally related and contain a conserved bioactive domain comprising six or seven canonical cysteine residues which are responsible for the characteristical three-dimensional "cystine-knot" motif of these proteins.

The various superfamily members can be further classified into distinct subfamilies and -groups, based on the extent of the homology/identity of their cystine-knot motif. The family of bone morphogenetic proteins (BMPs) is known to play a diverse set of roles in the musculoskeletal system and other tissues (see i.e. Ducy and Karsenty 2000, Kidney Int. 57, 2207-2214 for a review). Growth/differentiation factors (GDFs) are a distinct subgroup of the BMPs. Especially human GDF-5 (the protein is also known as MP52, CDMP-1 or sometimes as BMP-14), GDF-6 (CDMP-2, BMP13) and GDF-7 (CDMP-3, BMP-12) have been grouped together by several authors due to their comparable biological properties and the extraordinarily high degree of amino acid sequence identity (see i.e. Mikic 2004, Annals of Biomedical Engineering 32, 466-476; Wolfman et al. 1997, J. Clin. Invest. 100, 321-330).

Several diseases and genetic disorders which are related to the BMP/GDF signalling pathway have previously been elucidated. Besides loss-of-function disorders with reduced bone formation there is also a broad range of BMP-related diseases which in contrast lead to excessive ossification, calcification and/or the transformation of muscle into hard tissue mass.

A characteristic example of a disorder characterized by excessive ossification is fibrodysplasia ossificans progressiva (FOP). Individuals with FOP phenotype develop an extra skeleton due to the transformation of fibrous tissue. However, there are several other similar diseases such as e.g. sclerosterosis and progressive osseous heteroplasia (POH).

Hence, there is a great demand to develop improved drugs and therapies for the treatment of disorders characterized by excessive ossification and/or muscular degradation or -transformation.

This object is solved according to the invention by providing growth factor mutants which are able to antagonize the action of bone morphogenetic proteins.

Some frequently used terms herein are defined and exemplified as follows:

The term "bone morphogenetic proteins" or BMPs as used herein means the subclass of TGF-ß superfamily proteins generally known as bone morphogenetic proteins (BMPs). BMPs have been identified to exhibit osteogenic, chondrogenic and/or other growth and differentiation type activities. These BMPs (some are also known as GDFs) include e.g. BMP-2 (BMP-2A), BMP-3 (BMP-3A), BMP-4 (BMP-2B), BMP-5, BMP-6, BMP-7 (QP-1), BMP-8A (OP-2), BMP-8B (OP-3), BMP-9 (GDF-2), BMP-10, BMP-11 (GDF-11), BMP-12 (GDF-7), BMP-13 (GDF-6), BMP-14 (GDF-5), BMP-15 (GDF-9B), BMP-16, BMP-17, BMP-18, GDF-1, GDF-3, GDF-8 (Myostatin) and GDF-9 which sequences are all disclosed and well known to the person skilled in the art. For example, BMP-2, BMP-3, BMP-4, BMP-5, BMP-6 and BMP-7 are disclosed in United States Patents 5,108,922; 5,013,649; 5,116,738; 5,106,748; 5,187,076; and 5,141,905; BMP-8 is disclosed in PCT publication W091/18098; BMP-9 in PCT publication W093/00432, BMP-10 in United States Patent 5,637,480; BMP-11 in United States Patent 5,639,638, BMP-12 and BMP-13 are disclosed in United States Patent 5,658,882, BMP-15 is disclosed United States Patent 5,635,372, BMP-16 is disclosed in copending patent application serial number 08/715,202. Also included are any of the growth and differentiation factors (GDFs), including those described in PCT applications W094/15965; W094/15949; W095/01801; W095/01802; W094/21681; W094/15966; W095/10539; W096/01845; W096/02559. Also included is MP52, disclosed in PCT applications W093/16099 and WO96/33215 and monomeric MP52, disclosed in W099/61611 and WO01/11041.

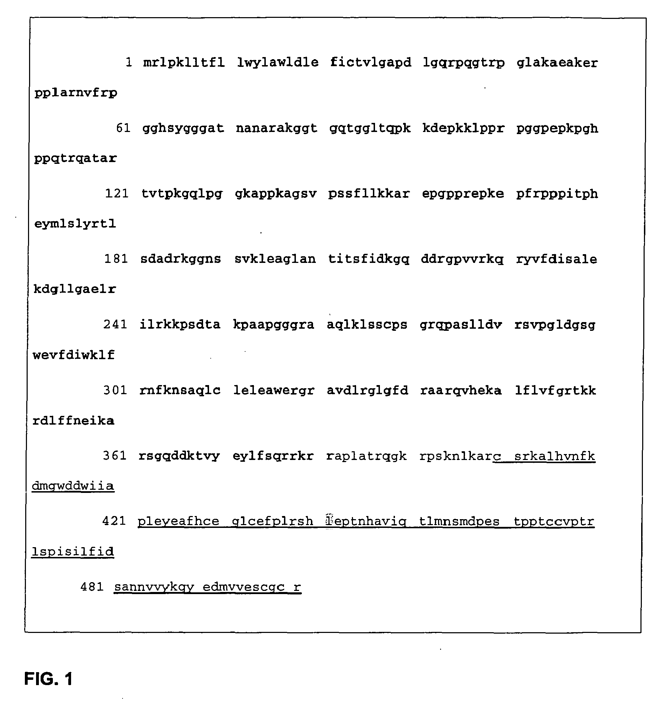

The term "cystine-knot-domain" as used herein means the well known and conserved cysteine-rich amino acid region which is present in the mature parts of TGF-beta superfamily proteins such as BMPs and GDFs and which forms a three-dimensional protein structure known as cystine-knot. In this domain, the respective location of the cysteine residues to each other is important and is only allowed to vary slightly in order not to lose the biological activity. Consensus sequences for cystine-knot domains are known in the state of the art. According to the definition defined herein the cystine-knot-domain of a protein starts with the first cysteine residue participating in the cystine-knot of the respective protein and ends with the residue which follows the last cysteine participating in the cystine-knot of the respective protein. For example, the cystine-knot domain of the human GDF-5 precursor protein (SEQ ID NO 1) comprises the amino acids 400-501 (see also Fig. 1).

The term "GDF-5-related protein" as used herein means any naturally occurring or artificially created bone morphogenetic protein which comprises a cystine-knot-domain with an amino acid identity of at least 40%, preferably at least 50%, more preferably at least 75% and most preferably at least 90% to the 102 aa cystine-knot domain of human GDF-5 (amino acids 400-501 of Fig. 1/SEQ ID NO 1). The percentage of identical residues can be easily determined by alignment of two sequences as described herein, followed by counting of the identical residues.

The term "proline-mutant" as used herein means a recombinant protein derived from a bone morphogenetic protein, a GDF-5-related protein or a variant thereof in which, after alignment with human GDF-5 as described in this application, the leucine, valine or methionine residue equivalent to leucine 441 (L441) of human GDF-5 (SEQ ID NO 1) has been replaced by proline (P).

The term "variant" as used herein means any of the following polypeptides:
a) biologically active fragments of a protein
b) protein constructs which contain additional sequences in excess to the original sequence of the protein
c) any combination of a) and b)

Biologically active fragments are meant to encompass parts of the known proteins which retain at least the amino acid positioned at 441 in GDF-5 or the equivalent position in other proteins. Such fragment is considered biologically active if at least one of the biological activities of the corresponding mature protein is still observable using known tests, preferably if a bone and/or cartilage inducing activity can be determined.

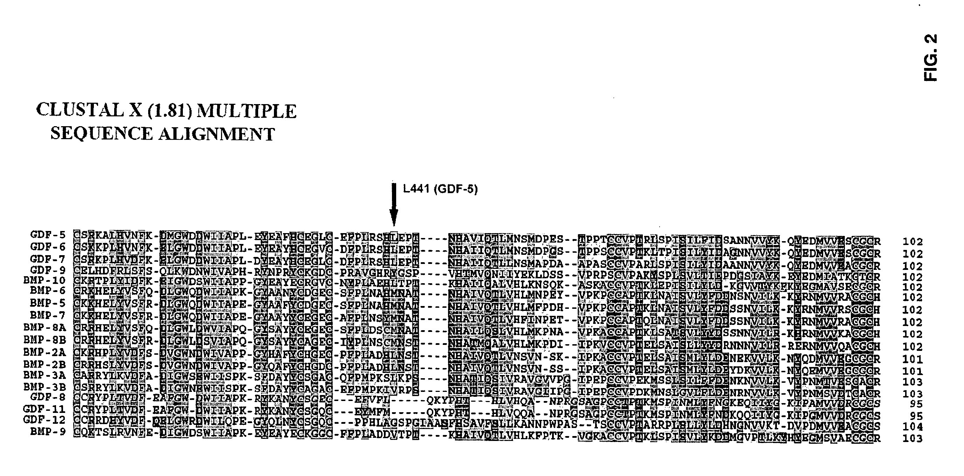

Due to the abolishment of osteogenic protein activity, BMP mutants are often characterized by reduced ossification and shortened or missing bones. For example, null mutants of GDF-5/CDMP-1/BMP-14 cause skeletal shortening of fingers and toes (brachydactyly type C), brachypodism in animals (Storm et al. 1994, Nature 368, 639-643) and acromesomelic dysplasias in man (Thomas et al. 1996, Nature Gen. 12, 315-317, Schwabe et al. 2004, Amer. J. Med Genet. 124A, 356-363). It has recently been discovered that disorders characterized by fibular hypoplasia and complex brachydactyly (DuPan syndrome) are presumably linked to the lack of active osteogenic GDF-5 protein. In individuals with DuPan syndrome, a L441P leucine-to-proline "loss-of-function" mutation at position 441 of human GDF-5 has been identified (Faiyaz-Ul-Haque et al. 2002, Clin. Genet. 61, 454-458). The finding that introduction of the strong protein helix and beta-strand breaker proline at a position equivalent to pos. 441 of GDF-5 is incompatible with the normal protein function is also supported by the fact that no native BMP molecule is carrying a proline residue (FIG. 2) at that specific position.

However, it has now surprisingly been found by means of functional studies and other experiments that in BMPs, the substitution of a leucine, valine or methionine residue with a proline residue at a position equivalent to pos 441 (leucine) of human GDF-5 generates completely unexpected novel protein functions which can be used e.g. for medicinal applications. According to this invention, recombinant bone morphogenetic protein mutants having a proline residue at this specific position exhibit antagonistic properties against bone morphogenetic proteins and are capable to modulate cell differentiation towards a muscular phenotype. These proteins can thus be efficiently used for scientific and therapeutic purposes and are e.g. suitable for the supportive therapy of muscular diseases, for the treatment of BMP-related disorders characterized by the excessive action of BMPs/GDFs, and for the modulation of stem cell differentiation towards a muscular phenotype.

The invention is further illustrated by FIG. 1-5.

FIG. 1 shows the human GDF-5 precursor protein (Hötten et al. 1994, Biochem. Biophys Res. Commun. 204, 646-652) which consists of a 381 aa prodomain (aa 1-381 including signal peptide part aa 1-27, bold letters) and a 120 aa mature part (aa 382-501). The mature part or especially the cystine-knot-domain (aa 400-501, underlined) are sufficient for the biological function of the protein. Residue L441 (grey box) is located within this cystine-knot domain.

Amino acid residues equivalent to L441 in the cystine-knot-domains of other bone morphogenetic proteins are shown in FIG. 2 (column marked by arrow). Substitution of a leucine, valine or methionine residue with proline results in generation of proteins with novel antagonistic and cell differentiation properties according to the present invention. Corresponding residues in bone morphogenetic proteins and variants thereof not shown in these figures can be easily determined by a sequence alignment with human GDF-5 as described in this patent application.

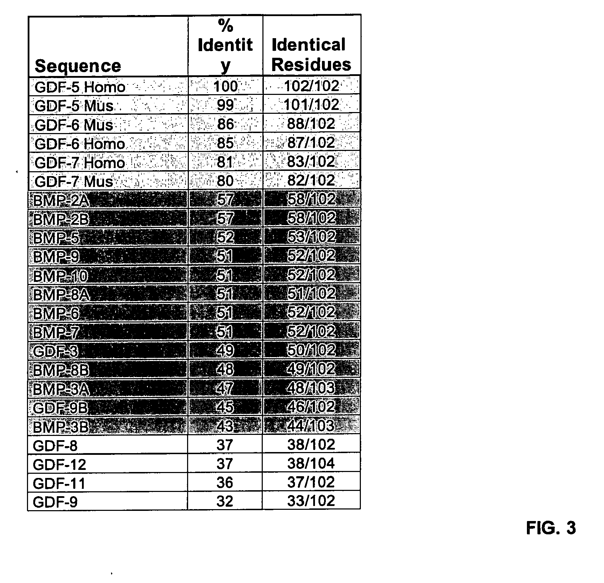

FIG. 3 shows a table with the sequence identities of cystine-knot-domains of known BMPs and GDFs to the cystine-knot-domain of human GDF-5. The sequences are available in patent applications or in the "Entrez" NCBI protein database (hftp://www.ncbi.nlm.nih.gov/Entrez/) under the following accession numbers: GDF-5 Homo: P43026, GDF-5 Mus: NP_032135, GDF-6 Mus: P43028, GDF-7 Homo: Q7Z4P5, GDF-7 Mus: P43029, GDF-7 Mus: BMP-2A: P12643, BMP-2B: P12644, BMP-9: Q9UK05, BMP-10: 095393, GDF-3: Q9NR23, GDF-8: 014739, GDF-11: 095390, GDF-9: NP_005251, GDF-9B: 095972, GDF-12: WO96/02559 (Patent),BMP-3A: P12645,BMP-3B: P55107,BMP-5: P22003,BMP-6: P22004,BMP-7: P18075, BMP-8A: P34821,BMP-8B: AAB77385.

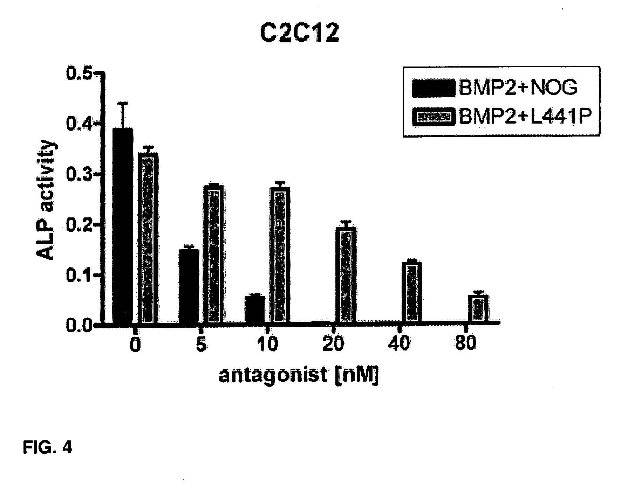

FIG. 4 shows a typical example of the antagonistic activity of proline-mutants according to the invention. Like several BMPs, recombinant BMP2 causes a characteristic induction of ALP activity in C2C12 progenitor cells. Induction of ALP is an established test system for the detection of osteogenic and chondrogenic properties of bone morphogenetic proteins. According to FIG. 4, addition of increasing amounts of GDF-5 mutant L441 P results in significant dose-dependant inhibition of the BMP2-mediated ALP induction. The effect is qualitatively comparable to the action of known BMP antagonist Noggin.

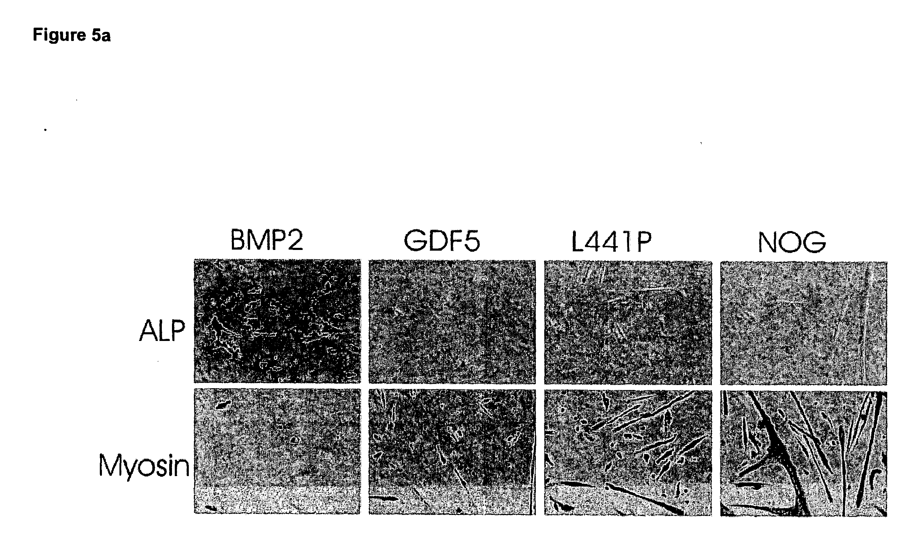

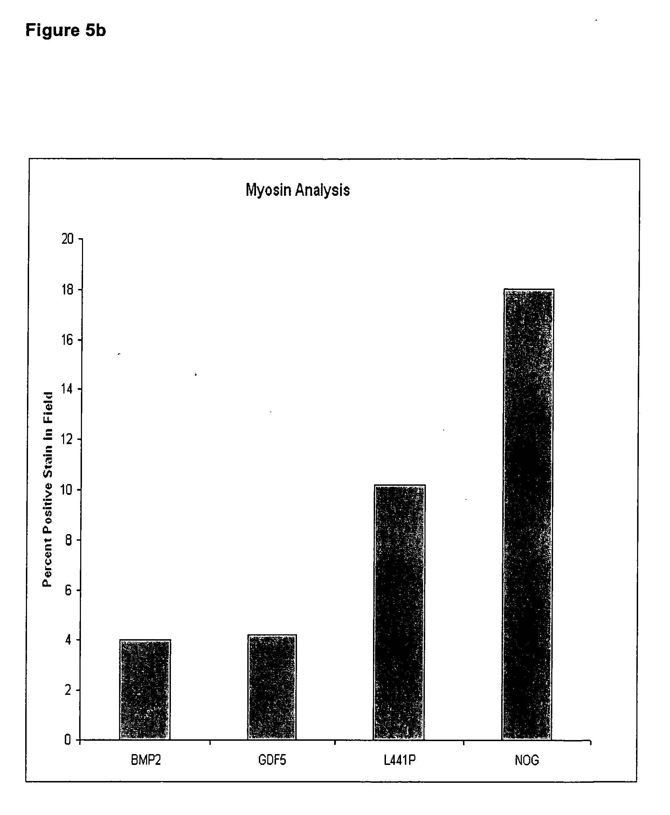

FIG. 5A and 5B exemplify the ability of proline-mutants to modulate myoblastic transdifferentiation. The process of myofibroblast transdifferentiation is characterized by the expression of muscle-specific genes such as alpha-actin and myosin. Differentiation of premyoblastic C2C12 cells was determined using ALP staining (osteoblastic lineage) or immunohistochemical analysis of myosin expression (muscle lineage). Treatment of C2C12 cells with BMP2 results in strong induction of ALP activity and suppression of muscle differentiation. Wildtype GDF5 has no effect on their spontaneous differentiation along the muscle lineage. In contrast, treatment with proline-mutant GDF-5 L441 P significantly increases muscle formation similar to the effects of the BMP inhibitor NOG (noggin), as indicated by the change of cell morphology and myosin expression.

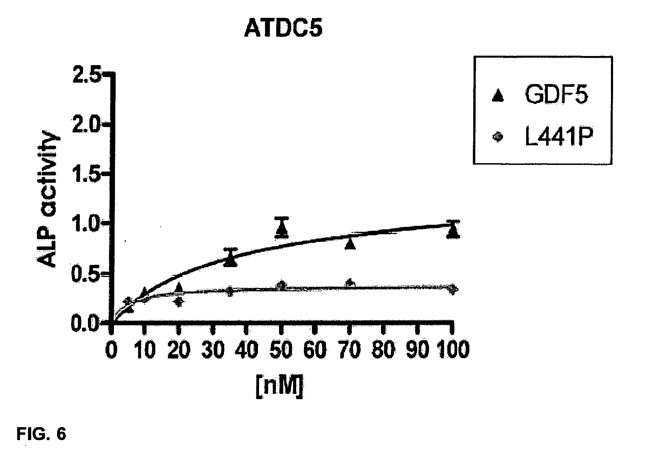

FIG.6 documents the loss of osteogenic activity of the proline-mutants. ALP activity was measured after stimulating chondrogenic ATDC5 cells with increasing amounts of recombinant proteins after 3 days. Recombinant wildtype GDFS displays characteristic dose-dependent inductions of ALP activity in ATDC5 cells. In contrast, GDF-5 mutant L441 P is almost inactive.

The antagonistic and thus antiosteogenic properties of the proline-mutants of the invention can especially be used for the therapy or prevention of several disorders characterized by deleterious calcification and/or excessive or ectopic ossification. Non limiting examples for such diseases are:

### Fibrodysplasia ossificans progressiva (FOP):

Fibrodysplasia ossificans progressiva, also known as myositis ossificans progressiva, is a genetically caused disease in which the body produces an extra skeleton. During the first or second decade of life, children form painful fibrous nodules over the neck, back, and shoulders which mature into bone in a process known as heterotopic ossification. FOP then progresses along the trunk and limbs of the body. These lesions slowly replace the body's muscles with normal appearing bone. Any attempt to remove the extra bone results in even more robust bone formation. Bone can be found in muscles, tendons, ligaments, and other connective tissues, therefore progressively restricting movement. To date, no effective cure exists to prevent the heterotopic ossification.

### Sclerosterosis:

Sclerosterosis is a progressive bone dysplasia that is characterized by hyperostosis and sclerosis, leading to thickening and deformation of skull, mandible, ribs, clavicles, and all other long bones.

### Progressive osseous heteroplasia (POH)

Progressive osseous heteroplasia is an extremely rare disorder characterized by abnormal development of bone in areas of the body where bone is not normally present. The disorder first appears as areas of patchy ossification on the skin. Ossification progresses to involve connective tissues, muscles, tendons, ligaments and fibrous tissue bands supporting muscles.

### Osteoma cutis

Osteoma cutis has a pathophysiology of bone arising in skin and soft tissues through mesenchymal ossification in the absence of an associated lesion.

### Myositis ossificans

Myositis ossificans is an aberrant reparative process that causes benign extraskeletal ossification in soft tissue and manifests in two forms: Myositis ossificans circumscripta which develops in response to trauma, injury, ischemia or inflammation; and myositis ossificans progressive which is a genetic disorder resulting in overexpression of BMP-4.

Further examples of disorders are neurotic osteosis, ectopic ossification caused by stress of operation, ossification of heart valves, ossification by defect of oxygen supply, osteogenic tumor, arterial sclerosis, ossification of the posterior longitudinal ligament (OPLL), pseudomalignant heterotopic ossification (PHO), pseudomalignant osseous tumor

According to the novel muscle-promoting properties of the proline-mutants as disclosed herein, the proteins of the invention can also be efficiently used for the treatment or prevention of diseases associated with muscular degeneration characteristics. Non limiting examples of such disorders are various neuromuscular diseases, cardiac insufficiency, weakness of single muscles such as e.g. the constrictor or bladder muscle, hypo- or hypertension caused by problems with the constrictor function of vascular smooth muscle cells, impotence/erectile dysfunction, incontinence, AIDS-related muscular weakness, amyotrophic lateral sclerosis (ALS), general and age-related amyotrophia. Especially preferred is the treatment of neuromuscular diseases which are aligned with joint or skeletal deformities. Non limiting examples for such disorders are Duchenne muscular dystrophy (DMD), Emery-Dreifuss muscular dystrophy (EDMD), congenital muscular dystrophy (CMD), intermediate spinal muscular atrophy (SMA or SMA2) and Charcot-Marie-Tooth disease (CMT).

As shown in the examples, the proline-mutants according to the invention are able to mimic the effects of known BMP inhibitors such as noggin. Thus, they are especially useful for prevention or therapy of a disorder characterized by insufficient action or lack of one or more bone morphogenetic protein antagonists. Examples for such bone morphogenetic protein antagonists which can be substituted by proline-mutants are noggin, chordin, ventropin, follistatin, dan, cerberus, protein related to dan and cerberus (PRDC), dante, caronte, sclerostin, tsg, gremlin and follistatin-related gene (FLRG). These and other bmp antagonists are known in the art and are e.g. disclosed in Canalis et al., Endocrine Reviews 24, 21-235 (2003).

In general, the proline-mutants according to the inventions can be used for the regulation of BMP-related signalling pathways and for prevention or therapy of a disorder in connection with unusual high BMP expression. Such disorders may cause deleterious and/or excessive formation of tissues and organs, such as e.g. bone, cartilage, connective tissue, connective tissue attachment, tendon, ligament, spinal/intervertebral disk, meniscus, blood vessels, dental tissue, dentin, periodontal ligament, skin, hair or neural tissue.

BMPs such as GDF-5, BMP2 or BMP7 (OP-1) are known for their potential to induce angiogenesis resulting in the formation of blood vessels (see e.g. Deckers et al. 2002, Endocrinology 143 (4), 1545-1553, Ramoshebi & Ripamonti 2000, The Anatomical Record 259, 97-107, Yamashita et al. 1997, Exp Cell Res. 235, 218-226). Some disorders are known, wherein the excessive formation of blood vessels is deleterious. One example is cancer, wherein angiogenesis results in increased tumor growth. In WO 02/054940 it is shown, that BMP2 caused an increase in the number of blood vessels in tumors from nude mice injected with 549 cells. Further examples are ophthalmic neovascularization which is a complication in a variety of ocular disorders and can lead to blindness.

Finally, the proline-mutants according to the invention are useful for the modulation of cell differentiation in vitro and in vivo, especially for the development of a muscular phenotype.

The biological activities of bone morphogenetic proteins and proline-mutants thereof i.e. in the field of induction of bone, cartilage and connective tissue such as i.e. periodontal ligament can be easily determined with the help of established test systems. Most useful and preferred is a common *in vitro* test known as alkaline phosphatase (ALP) assay (Takuwa et al. 1989, Am. J. Physiol. 257, E797-E803), which is demonstrated in example 2 / FIG. 6. GDF-5-related proteins have been demonstrated to increase alkaline phosphatase activity i.e. in ROB-C26 osteoprogenitor cells (Yamaguchi et al. 1991, Calcif. Tissue Int. 49, 221-225) as described in W095/04819, in embryonic ATDC5 cells (Riken Gene Bank, ROB 0565), in mouse stromal MCHT-1/26 cells, and in periodontal ligament (HPDL) cells as shown in Nakamura et al. 2003, J. Periodontal Res. 38,597-605.

The determination of corresponding amino acid positions in related amino acid sequences as well as the calculation of percentages of identity can be performed with the help of well known alignment algorithms and optionally computer programs using these algorithms. The amino acid identities in this patent application have been calculated by aligning sequences with the freeware program ClustaIX (Version 1.81) with default parameters and subsequent counting of identical residues by hand. Default settings for pairwise alignment (slow-accurate) are: gap opening parameter: 10.00; gap extension parameter 0.10; Protein weight matrix: Gonnet 250. The ClustalX program is described in detail in:
Thompson,J.D., Gibson,T.J., Plewniak,F., Jeanmougin,F. and Higgins,D.G. (1997) The ClustalX windows interface: flexible strategies for multiple sequence alignment aided by quality analysis tools. Nucleic Acids Research 24:4876-4882.
ClustaIX is a windows interface for the ClustaIW multiple sequence alignment program and is i.e. available from various sources, i.e. by anonymous ftp from ftp-igbmc.u-strasbg.fr, ftp.embl-heidelberg.de, ftp.ebi.ac.uk or via download from the following webpage: http://www-igbmc.u-strasbg.fr/Biolnfo/. The ClustaIW program and algorithm is also described in detail in:
   Thompson, J.D., Higgins, D.G. and Gibson, T.J. (1994) CLUSTALW: improving the sensitivity of progressive multiple sequence alignment through sequence weighting, positions-specific gap penalties and weight matrix choice. Nucleic Acids Research 22:4673-4680.

The GDF-5-related proteins as defined herein comprise a cystine-knot-domain with an amino acid identity of at least 40%, preferably at least 50%, more preferably at least 75% and most preferably at least 90% to the 102 aa cystine-knot domain of human GDF-5 (amino acids 400-501 of Fig. 1/SEQ ID NO 1). According to FIG. 3, a limiting value of at least 40% identity excludes the GDF-8, GDF-9, GDF-11 and GDF-12 proteins which all do not have a leucine, valine or methionine residue at a position equivalent to pos 441 of human GDF-5 and are therefore not comprised in the invention. A limiting value of at least 75% identity is well suitable to separate the highly related members of the GDF-5/-6/-7 group of proteins and variants thereof from other BMPs. A limiting value of at least 90% identity delimits GDF-5 molecules of different species from other bone morphogenetic proteins.

In a preferred embodiment, the proline-mutant according to the invention is a proline-mutant of a GDF-5-related protein or a variant thereof and has the potential to antagonize the multiple actions of GDF-5-related proteins. It has been demonstrated that GDF-5-related proteins are important inducers and regulators/differentiators of i.e. bone and cartilage (Cheng et al. 2003, J. Bone & Joint Surg. Am. 85-A, 1544-1552; Settle et al. 2003, Developm. Biol. 254, 116-130), connective tissue such as tendon and ligament (Wolfman et al. 1997, J. Clin. Invest. 100, 321-330), nerve tissue (Farkas et al. 1997, Neurosci Lett. 236,120-122; Watakabe et al. 2001, J. Neurochem. 76, 1455-1464), stem cells (Shimaoka et al. 2003, J. Biomed. Materials Res. Part A 68A, 168-176; Bai et al. 2004, Biochem. Biophys. Res. Commun. 325, 453-460) and/ periodontal ligament and teeth (Sena et al 2003, J. Dent. Res. 82, 166-171; Morotome et al. 1998, Biochem. Biophys. Res. Commun. 244, 85-90).

Non-limiting examples of GDF-5 related proteins according to the definition above (90% identity limitation) are human GDF-5 (disclosed as MP52 in WO95/04819 and in Hötten et al. 1994, Biochem. Biophys Res. Commun. 204, 646-652), recombinant human GDF-5/MP52 (WO96/33215), mouse GDF-5 (US 5,801,014), CDMP-1 (WO96/14335), HMW human MP52s (WO97/04095), rabbit GDF-5 (Sanyal et al. 2000, Mol Biotechnol. 16, 203-210), human GDF-6/BMP-13 (US 5,658,882), bovine GDF-6 (NCBI accession no P55106), mouse GDF-6 (NCBI accession no NP_038554), GDF-6/CDMP-2 (WO96/14335), human GDF-7/BMP-12 (US 5,658,882), mouse GDF-7 (NCBI accession no AAP97721), GDF-7/CDMP-3 (WO96/143335), chicken GDF-5 (NCBI accession no. NP_989669), monomeric GDF-5, -6 and -7 (WO 01/11041 and WO99/61611), Growth factor mutants with improved biological activity (Eur. Pat. Appl. No. 05 004 840.4).

In a more preferred embodiment, the proline-mutant protein according to the invention is a proline-mutant of a vertebrate or recombinant GDF-5 protein or a variant thereof and has the potential to antagonize the multiple actions of GDF-5 and other bone morphogenetic proteins. Native GDF-5 is considered to be a very effective promoter of bone and cartilage formation as well as connective tissue formation (see for example WO 95/04819, Hötten et al. 1996, Growth Factors 13, 65-74; Storm et al. 1994, Nature 368, 639-643; Chang et al. 1994, J. Biol. Chem. 269, 28227-28234) and formation of connective tissue attachment (EP 0 831 884). In this context, GDF-5 is useful for applications concerning the joints between skeletal elements (see for example Storm & Kingsley 1996, Development 122, 3969-3979). One example for connective tissue is tendon and ligament (Wolfman et al. 1997, J. Clin. Invest. 100, 321-330; Aspenberg & Forslund 1999, Acta Orthop Scand 70, 51-54; WO 95/16035). The protein is helpful for meniscus and spinal/intervertebral disk repair (Walsh et al. 2004, Spine 29, 156-63) and spinal fusion applications (Spiro et al. 2000, Biochem Soc Trans. 28, 362-368). GDF-5 can be beneficially applied in tooth (dental and periodontal) applications (see for example WO 95/04819; WO 93/16099; Morotome et al. 1998, Biochem Biophys Res Comm 244, 85-90) such as the regeneration of dentin or periodontal ligament. GDF-5 is also useful in wound repair of any kind. It is also beneficial for promoting tissue regeneration in the neuronal system and survival of e.g. dopaminergic neurons. In this context, GDF-5 can be used for treating neurodegenerative disorders like e.g. Parkinson's disease and possibly also Alzheimer's disease or Huntington chorea tissues (see for example WO 97/03188; Krieglstein et al., (1995) J. Neurosci Res. 42, 724-732; Sullivan et al., (1997) Neurosci Lett 233, 73-76; Sullivan et al. (1998), Eur. J. Neurosci 10, 3681-3688). GDF-5 allows to maintain nervous function or to retain nervous function in already damaged tissues. GDF-5 is therefore considered to be a generally applicable neurotrophic factor. It is also useful for diseases of the eye, in particular retina, cornea and optic nerve (see for example WO 97/03188; You et al. (1999), Invest Opthalmol Vis Sci 40, 296-311), for hair growth and the treatment and diagnosis of skin related disorders (WO 02/076494; Battaglia et al. 2002, Trans. Orthop. Res. Soc. 27, 584), and for induction of angiogenesis (Yamashita et al. 1997, Exp. Cell Res. 235, 218-26). Examples for GDF-5 proteins and variants thereof are: human GDF-5 (disclosed as MP52 in WO95/04819 and as human GDF-5 in Hötten et al. 1994, Biochem. Biophys Res. Commun. 204, 646-652), recombinant human GDF-5/MP52 (WO96/33215), HMW human MP52s (WO97/04095), CDMP-1 (WO96/14335), mouse (Mus musculus) GDF-5 (US 5,801,014), rabbit (Oryctolagus cuniculus) GDF-5 (Sanyal et al. 2000, Mol Biotechnol. 16, 203-210), chicken (Gallus gallus) GDF-5 (NCBI accession no. NP_989669), african clawed frog (Xenopus laevis) GDF-5 (NCBI accession no. AAT99303), monomeric human GDF-5 (WO 01/11041 and WO99/61611).

Enclosed in these embodiments are also proline-mutants of allelic versions of the aforementioned genes/proteins as well as proline-mutants of the vertebrate, mammalian and recombinant proteins or variants thereof having additional mutations such as substitutions, additions and deletions, as long as these additional mutations have no essential effect on protein activity.

The proline-mutants according to the invention can be easily produced in various prokaryotic and eukaryotic expression systems, in particular by expression in prokaryotes and subsequent renaturation/refolding according to known methods (see i.e. WO96/33215).

A further subject matter of the present invention is a nucleic acid encoding a proline-mutant according to the invention. The nucleic acid has a sequence such that a substitution of the leucine, valine or methionine residues equivalent to leucine 441 of human GDF-5 with a proline is achieved. The base triplets coding for these amino acids and the degeneracy of the genetic code are generally known. Possible base triplets for proline are CCT (CCU), CCC, CCA and CCG. The nucleic acid can be a DNA sequence and/or a RNA sequence, as long as the protein according to the invention can be obtained from this nucleic acid upon expression in a suitable system.

Expression vectors are a further subject matter of the present invention, wherein the nucleic acid is inserted in a suitable vector system, the vector system being selected according to the desired expression of the protein. The vector system can be a eukaryotic vector system, but preferred is a prokaryotic vector system, with which the proteins can be produced in a particularly easy and pure manner. A suitable expression vector is i.e. shown in WO96/33215. The expression vector can also be a viral vector which can be used i.e. in gene therapy approaches.

Host cells are also a subject matter of the present invention. The host cells are characterized in that they contain a nucleic acid or an expression vector according to the invention and that they are able to use the information present in the nucleic acids and in the expression vector, respectively, for the expression of proline-mutants according to the invention. Suitable host cells are preferably prokaryotic cells, in particular most *E. coli* strains. Particularly useful host strains are descendents of *E. coli* W3110 as shown e.g. in WO96/33215. In a preferred embodiment, host cells, preferably of human origin, may also be useful for transplantation to patients in need thereof.

Another subject matter of the present invention are antibodies against proline-mutants. These antibodies according to the present invention are specific for the claimed recombinant proline-mutant. These antibodies according to the present invention can be generated by using those fragments of the protein of the invention as described above as immunogens to generate antibodies by known methods. The antibodies can be monoclonal or polyclonal and they can be of any isotype. Also comprised are antibody fragments such as Fab-fragments or Fab₂-fragments. The antibodies can also be humanized antibodies or chimeric antibodies etc. These antibodies are useful e.g. for purification purposes or diagnostic purposes such as monitoring the proline-mutant in body fluids of patients which are either diagnosed with Du Pan syndrome/brachydactyly A2 or which are treated with the proline-mutant or host cells expressing the proline mutant.

Further subject matters of the present application are pharmaceutical and/or diagnostic compositions comprising at least one proline-mutant of a bone morphogenetic protein or a nucleic acid or a vector or host cell according to the invention. Suitable are generally all pharmaceutical compositions which have already been published in context with bone morphogenetic proteins. An expression vector or a host cell can be considered to be advantageous as active substances in a pharmaceutical and/or diagnostic composition. Also combinations of a protein according to the invention with other proteins can be used in preferred pharmaceutical compositions. Combinations with other proteins such as other BMP antagonists but also with growth factors as i.e. NGF, BDNF, EGF, PDGF, NT-3, -4,-5, chordin and/or hedgehog proteins are possible. Of course this invention also comprises pharmaceutical compositions containing further substances like e.g. pharmacologically acceptable auxiliary and carrier substances. The formulation may include antioxidants, preservatives, colouring, flavouring and emulsifying agents, suspending agents, solvents, fillers, bulking agents, buffers, delivery vehicles, excipients and/or pharmaceutical adjuvants. For example, a suitable carrier or vehicle may be water for injection, physiological saline solution, or a saline solution mixed with a suitable carrier protein such as serum albumin. A preferred antioxidant for the preparation of the composition of the present invention is ascorbic acid.

Cosmetic compositions known in the art, preferably hypoallergic and pH controlled are also useful within the context of the present invention, and include toilet waters, packs, lotions, skin milks or milky lotions. Said preparations contain, besides the active compound, components usually employed in such preparations. Examples of such components are oils, fats, waxes, surfactants, humectants, thickening agents, antioxidants, viscosity stabilizers, chelating agents, buffers, preservatives, perfumes, dyestuffs, lower alkanols, and the like. If desired, further ingredients may be incorporated in the compositions, e.g. antiinflammatory agents, antibacterials, antifungals, disinfectants, vitamins, sunscreens, antibiotics, or other anti-acne agents.

The solvent or diluent of the pharmaceutical composition may be either aqueous or non-aqueous and may contain other pharmaceutically acceptable excipients which are capable of modifying and/or maintaining a pH, osmolarity, viscosity, clarity, scale, sterility, stability, rate of dissolution or odour of the formulation. Similarily other components may be included in the pharmaceutical composition according to the present invention in order to modify and/or maintain the rate of release of the pharmaceutically effective substance. Such modifying components are substances usually employed in the art in order to formulate dosages for parenteral administration in either unit or multi-dose form. The finally formulated pharmaceutical and/or diagnostic composition prepared according to the present invention may be stored in sterile vials in form of a solution, suspension, gel, emulsion, solid or dehydrated or lyophilized powder. These formulations may be stored either in a ready-to-use form or in a form, e.g. in case of a lyophilized powder, which requires reconstitution prior to administration. The above and further suitable pharmaceutical formulations are known in the art and are described in, for example, Gus Remington's Pharmaceutical Sciences (18th Ed., Mack Publishing Co., Eastern, Pa., 1990, 1435-1712). Such formulations may influence the physical state, stability, rate of in vivo release and rate of in vivo clearance of the pharmaceutically effective component. Other effective administration forms comprise parenteral slow-release, i.e. retarded, formulations, inhalent mists, or orally active formulations. For example, a slow-release formulation may comprise proteins bound to or incorporated into particulate preparations of polymeric compounds (such as polylactic acid, polyglycolic acid, etc.) or liposomes. The pharmaceutical composition according to the present invention may also be formulated for parenteral administration, e.g., by infusion or injection, and may also include slow-release or sustained circulation formulations. Such parenterally administered therapeutic compositions are typically in the form of pyrogen-free, parenterally acceptable aqueous solutions comprising the pharmaceutically effective component(s) in a pharmaceutically acceptable carrier and/or diluent. The pharmaceutical composition may comprise a matrix material, i.e. in cases where local application, e.g. at a site of ectopic bone formation, is intended. It is advantageous to the protein, the nucleic acid, the expression vector or the host cell when they are applied in and/or on a biocompatible matrix material. Matrix material as used herein means a carrier or matrix acting as a scaffold for cell recruitment, attachment, proliferation and differentiation and/or as a potential delivery and storage device for proline-mutants of bone morphogenetic proteins. In contrast to the solid matrices, carriers consist of amorphous materials having no defined surfaces and lacking a specific shape, i.e. alkylcelluloses, pluronics, gelatins, polyethylene glycols, dextrins, vegetable oils, sugars and other liquid and viscous substances.

Uses of bone morphogenetic proteins in combination with matrix materials are extensively published and described, such as for example in WO98/21972. These matrix materials are equally suitable for proline-mutants according to the invention. The matrix material can be transplanted into the patient, e.g. surgically, wherein the protein or the DNA encoding the protein can be slowly released from the matrix material and then be effective over a long period of time. All types of matrix materials are useful in accordance with the present invention, as long as they are biocompatible and selected for the intended area or indication of use. The matrix material can be a natural material, a modified natural material as well as a synthetic material. All already known matrices for morphogenetic proteins are encompassed. Examples of natural materials are e.g. autologous, heterologous or xenologous bone materials, collagen, e.g. collagen type I and iii, or metals like titanium. Also other components of the extracellular matrix can be used. The extracellular matrix comprises for example the various collagens, as for example types I, II, V, IX, X, Xi and XIII, further proteoglycanes and glycosaminoglycanes, as for example chondroitinsulfate, biglycane, decorine and/or hyaluronic acid, or noncollagenous proteins as for example osteopontin, laminin, fibronectin, vitronectin, thrombospondin, cartilage matrix protein and dentin phosphoprotein. Preferred materials derive from muscle tissue. All mentioned natural materials may also be used in artificially modified forms. Examples of modified natural materials are demineralized bone, thermoashed bone mineral, sintered bone or chemically crosslinked hyaluronic acid (hydrogel), or metal alloys. Examples of synthetic materials are polymers like polyglycolic acid, polylactide and polylactide derivatives such as e.g. polylactic acid, poly(lactide-co-glycolide), polylactid acid-polyethylene glycol or glycolide L-lactide copolymers, further polyphosphates, polyethylene glycol, polyoxyethylene polyoxypropylene copolymers or materials containing calcium phosphates such as beta-tricalcium phosphate (Ca₃(PO₄)₂), alpha-tricalcium phosphate and hydroxyl apatite. Further examples of other useful matrix materials belonging to one of the above mentioned groups are Ca(OH)₂, coral, natural bone mineral, chitin, non-demineralized bone particles, ceramic bone particles, ceramic dentin, irradiated cancellous bone chips, plaster of Paris, bioactive glass, apatite-wollastonite-containing glass ceramic. Also a combination of the above mentioned carriers and/or matrices can form the matrix material as for example the combination of hydroxy apatite and collagen (e.g. Healos, previously available from Orquest, Inc., CA, USA, [now DePuy Acromed, MA, USA]), a combination of polyglycolic acid and polylactic acid or polylactid derivatives, or coral-collagen composites. For a non limiting list of useful carriers and matrices see further i.e. Kirker-Head 2000, Advanced Drug Delivery 43, 65-92.

SEQ ID NO 1 shows the DNA and protein sequence of the human GDF-5 precursor. In the preferred human GDF-5 protein mutant with improved bioiogicai acitivity, the leucine residue at pos 441 is substituted with proline.

SEQ ID NO 2 shows the DNA and protein sequence of the human monomeric GDF-5.

The following non limiting examples are intended to further illustrate the invention.

### Example 1: Preparation of recombinant proteins

For the expression of mutant GDF5, patient DNA carrying the mutant L441 P was amplified by PCR with specific GDF-5 primer pair F_MP52_Afl II (GCAAGAACCTTAAGGCTCGC) and R_MP52_Nco I (CGGGGTCCATGGAGTTCATC) with Pfx DNA Polymerase (Invitrogen). The amplified DNA was ligated into the protein expression vector pKOT277 and confirmed by DNA sequencing. For the expression of other BMP mutants, DNAs coding for the mature parts of bone morphogenetic proteins have been isolated from human ROB-C26 osteoprogenitor cells (Yamaguchi et al. 1991, Calcif. Tissue Int. 49, 221-225) via RT-PCR technique and subsequently ligated into prokaryotic plasmid vectors. Mutations were created by using the QuickChangeTM site-directed mutagenesis kit with the PfuTurboTm DNA polymerase and the DPN I endonuclease from Stratagene according to the instruction manual of the manufacturer. The proteins were expressed in inclusion bodies using the bacterial E. coli strain W3110BP and isolated using a homogenization buffer (25 mM Tris HCl, pH 7.3, 10 mM EDTA, pH 8.0) and wash buffer (1 M Urea, 20 mM Tris HCl, 5 mM EDTA, pH 8.3). After centrifugation the inclusion bodies were dissolved in 6 M Guanidin HCl, 50 mM Tris, 150 mM NaCl, 3 mM DTT, pH 8.0). Further purification was carried out on a reversed phase column Aquapore Octyl (Applied Biosys, CV = 7.8 ml, 100x10, 20µ, No 186470) with a gradient from 100% of Eluent A (0.1 % TFA) to 100% Eluent B (0.1 % TFA, 90 % CH3N) in 104 minutes (flow rate: 3 ml/min). Mutant proteins were dissolved in buffer (6 M Guanidin HCl, 50 mM Tris, 150 mM NaCl, 3 mM DTT, pH 8.0), the protein concentration adjusted to 2.6 mg/ml and the pH between 8 and 9. After 2 h incubation at room temperature, refolding buffer (1 M NaCl, 50 mM Tris, 5 mM EDTA, 1 mM GSSG, 2 mM GSH, 33 mM Chaps, pH 9.5) was added under gentle agitation to reach a final protein concentration of 0.16 mg/ml. The solution was then incubated for 48 h at 22° C and the refolding was stopped by changing the pH to 3-4 by adding HCl to 18% (vol/vol). After centrifugation, the non-refolded monomer was separated from the dimer form by carrying out a second RP-HPLC under the same conditions. Purity was controlled by a western blot and SDS PAGE silver stain, the fractions containing the dimerized protein were pooled and lyophilized. Subsequently the proteins were dissolved in 10 mM HCl and stored at -80°C. BMP2 was prepared as previously described (Ruppert, R., Hoffmann, E., and Sebald, W. 1996. Human bone morphogenetic protein 2 contains a heparin-binding site which modifies its biological activity. Eur J Biochem 237:295-302.). Human recombinant NOG (noggin) was a kind gift from A. Economides (Regeneron Pharmaceuticals, Inc).

### Example 2: Alkaline phosphatase assay

Quantitative ALP assays were performed as described (Knaus, P., and Sebald, W. 2001. Cooperativity of binding epitopes and receptor chains in the BMP/TGFbeta superfamily. Biol Chem 382:1189-1195.) with minor modifications: ATDC5 and C2C12 cells were seeded at a density of 1x10⁴ (?) cells/96well in growth media (for ATDC5: DMEM:F12, 5% FCS, 10 µg/ml transferrin, 30 nM sodium selenite, 2 mM LGlutamine in 5% CO₂ - for C2C12: DMEM high glucose, 10% FCS, 2 mM L-Glutamine in 10% CO₂). After 24 h cells were starved for 5 h in media with reduced FCS (for ATDCS: 8 DMEM/F12, 0.5% FCS, 2 mM L-Glutamine in 5% CO₂ - for C2C12: DMEM high glucose, 2% FCS, 2 mM L-Glutamine in 10% CO₂). Stimulation with recombinant proteins was performed in starving media for 3 days. ALP activity was measured for ATDC5 and C2C12 cells directly in 96well plate by lysing cells in ALP-buffer1 (0.1 M Glycine, pH 9.6, 1% NP-40, 1 mM MgCl₂, 1 mM ZnCl₂). ALP activity of micromass cultures was determined by homogenizing cultures in ALP-buffer1 and after centrifugation only supernatant was used for further proceedings. After addition of one volume of ALP-buffer2 (5 mM pNPP, 0.1 M Glycine, pH 9.6, 1 mM MgCl₂, 1 mM ZnCl₂) alkaline phosphatase activity was determined spectrophotometrically. The amount of p-nitrophenyl released from the substrate p-nitrophenyl-phosphate was recorded at 405 nm and used to calculate ALP activity.

### Example 3: Cell differentiation assay

For differentiation of C2C12, cells were grown on coverslips in DMEM high glucose supplemented with 10% FCS, 2 mM L-Glutamine and Penicillin/Streptomycin. Before reaching confluence cells were stimulated with 10 nM recombinant proteins in DMEM high glucose supplemented with only 2% FCS, 2 mM L-Glutamine and Penicillin/Streptomycin over 5 days. ALP staining was performed to visualize osteoblastic differentiation. Myoblastic differentiation was analyzed by immuncytochemistry using an antibody against myosin heavy chain (anti-Myosin-IgG mouse, SIGMA, 1:300). Detection was achieved after extensive washings by Vectastain-Kit (Vector) and DAB-staining (Vector) according to the manufacturer's recommendations.

## Claims

1. A recombinant protein which is derived from a bone morphogenetic protein or a variant thereof by converting a hydrophobic leucine, valine or methionine residue at a position equivalent to leucine 441 of human wildtype GDF-5 (SEQ ID NO 1) to a proline residue.

2. The recombinant protein according to claim 1 wherein said recombinant protein is derived from the group consisting of mammalian BMP-2 (BMP-2A), BMP-3 (BMP-3A), BMP-3B (GDF-10), BMP-4 (BMP-2A), BMP-5, BMP-6, BMP-7 (OP-1), BMP-8A (OP-2), BMP-8B (OP-3), BMP-9, BMP-10, BMP-12 (GDF-7), BMP-13 (GDF-6), BMP-14 (GDF-5), or variants thereof.

3. The recombinant protein according to claim 1 wherein said recombinant protein is derived from a GDF-5-related protein.

4. The recombinant protein according to claim 3 which is derived from dimeric human GDF-5 (SEQ ID NO 1) or monomeric human GDF-5 (SEQ ID NO 2), or variants thereof.

5. Nucleic acid, encoding a protein according to any one of the preceding claims.

6. Expression vector, comprising a nucleic acid according to claim 5.

7. Host cell, containing a nucleic acid according to claim 5 or an expression vector according to claim 6.

8. Antibody directed against a recombinant protein having antagonistic activity against bone morphogenetic proteins according to any one of claims 1 to 4.

9. Pharmaceutical composition, comprising a protein according to any one of claims 1 to 4, or/and a nucleic acid according to claim 5, or/and an expression vector according to claim 6, or/and a host cell according to claim 7, or/and an antibody according to claim 8.

10. Pharmaceutical composition according to claim 9, additionally comprising pharmacologically acceptable auxiliary and/or carrier substances.

11. Pharmaceutical composition according to claims 9 or 10,
wherein the protein and/or nucleic acid and/or vector and/or host cell are contained in and/or on a biocompatible matrix material.

12. Use of a recombinant protein having antagonistic activity against bone morphogenetic proteins according to any one of the claims 1 to 4, or use of a nucleic acid according to claim 5, or use of an expression vector according to claim. 6, or use of a host cell according to claim 7, or use of an antibody according to claim 8, or use of a pharmaceutical composition according to any one of claims 9 to 11, for
a) the preparation of a medicament for prevention or therapy of a disorder **characterized by** deleterious calcification and/or excessive or ectopic ossification
b) the preparation of a medicament for prevention or therapy of a disorder **characterized by** insufficient action or lack of one or more bone morphogenetic protein antagonists
c) the preparation of a medicament for prevention or therapy of a disorder **characterized by** muscular degradation or muscular weakness.
d) the preparation of a medicament for the prevention or therapy of a disorder in connection with BMP-caused deleterious formation of bone, cartilage, connective tissue, connective tissue attachment, tendon, ligament, spinal/intervertebral disk, meniscus, dental tissue, dentin, periodontal ligament, blood vessels, skin, hair or neural tissue. e) the preparation of a medicament for direction of cell differentiation towards a muscular phenotype.
f) the preparation of a medicament for regulation of BMP-related signalling pathways
g) the preparation of a medicament for the prevention or therapy of a disorder caused by unusual high expression of BMPs

13. Use according to claim 12 a), wherein said disorder is selected from the group consisting of neurotic osteosis, ectopic ossification caused by stress of operation, ossification of heart valves, ossification by defect of oxygen supply, osteogenic tumor, arterial sclerosis, ossification of the posterior longitudinal ligament (OPLL), pseudomalignant heterotopic ossification (PHO), pseudomalignant osseous tumor, myositis ossificans circumscripta, fibrodysplasia ossificans progressiva (FOP), osteoma cutis, progressive osseous heteroplasia and sclerosteosis.

14. Use according to claim 12 b) wherein the lacking or insufficiently working BMP antagonist is selected from the group consisting of Noggin, chordin, ventropin, follistatin, dan, cerberus, protein related to dan and Cerberus (PRDC), dante, caronte, sclerostin, tsg, gremlin and follistatin-related gene (FLRG)

15. Use according to claim 12 c) wherein said muscular disorder is selected from the group consisting of neuromuscular diseases, cardiac insufficiency, weakness of single muscles such as e.g. the constrictor or bladder muscle, hypo- or hypertension caused by problems with the constrictor function of vascular smooth muscle cells, impotence/erectile dysfunction, incontinence, AIDS-related muscular weakness, amyotrophic lateral sclerosis (ALS), general and age-related amyotrophia.

16. Use according to claim 15 wherein said neuromuscular disease is selected from the group consisting of Duchenne muscular dystrophy (DMD), Emery-Dreifuss muscular dystrophy (EDMD), congenital muscular dystrophy (CMD), intermediate spinal muscular atrophy (SMA or SMA2) and Charcot-Marie-Tooth disease.

17. Use according to claim 12 (d), wherein the deleterious blood vessel formation is caused by a cancer disorder.


SEQUENCE LISTING

<110> Biopharm und weitere

<120> Growth Factor Mutants with Improved Biological Activity

<130> 35586PEP

<140>
<141>

<160> 4

<170> PatentIn Ver. 2.1

<210> 1
<211> 2703
<212> DNA
<213> Homo sapiens

<220>
<221> CDS
<222> (640)..(2142)

<400> 1
ccatggcctc gaaagggcag cggtgatttt tttcacataa atatatcgca cttaaatgag 60

tttagacagc atgacatcag agagtaatta aattggtttg ggttggaatt ccgtttccaa 120

ttcctgagtt caggtttgta aaagattttt ctgagcacct gcaggcctgt gagtgtgtgt 180

gtgtgtgtgt gtgtgtgtgt gtgtgtgtga agtattttca ctggaaagga ttcaaaacta 240

gggggaaaaa aaaactggag cacacaggca gcattacgcc attcttcctt cttggaaaaa 300

tccctcagcc ttatacaagc ctccttcaag ccctcagtca gttgtgcagg agaaaggggg 360

cggttggctt tctcctttca agaacgagtt attttcagct gctgactgga gacggtgcac 420

gtctggatac gagagcattt ccactatggg actggataca aacacacacc cggcagactt 480

caagagtctc agactgagga gaaagccttt ccttctgctg ctactgctgc tgccgctgct 540

tttgaaagtc cactcctttc atggtttttc ctgccaaacc agaggcacct ttgctgctgc 600

cgctgttctc tttggtgtca ttcagcggct ggccagagg atg aga ctc ccc aaa 654

Met Arg Leu Pro Lys
1               5

ctc ctc act ttc ttg ctt tgg tac ctg gct tgg ctg gac ctg gaa ttc 702

Leu Leu Thr Phe Leu Leu Trp Tyr Leu Ala Trp Leu Asp Leu Glu Phe
                10                  15                  20

atc tgc act gtg ttg ggt gcc cct gac ttg ggc cag aga ccc cag ggg 750

Ile Cys Thr Val Leu Gly Ala Pro Asp Leu Gly Gln Arg Pro Gln Gly
            25                  30                  35

acc agg cca gga ttg gcc aaa gca gag gcc aag gag agg ccc ccc ctg 798

Thr Arg Pro Gly Leu Ala Lys Ala Glu Ala Lys Glu Arg Pro Pro Leu
        40                  45                  50

gcc cgg aac gtc ttc agg cca ggg ggt cac agc tat ggt ggg ggg gcc 846

Ala Arg Asn Val Phe Arg Pro Gly Gly His Ser Tyr Gly Gly Gly Ala
    55                  60                  65

acc aat gcc aat gcc agg gca aag gga ggc acc ggg cag aca gga ggc 894

Thr Asn Ala Asn Ala Arg Ala Lys Gly Gly Thr Gly Gln Thr Gly Gly
70                  75                  80                  85

ctg aca cag ccc aag aag gat gaa ccc aaa aag ctg ccc ccc aga ccg 942

Leu Thr Gln Pro Lys Lys Asp Glu Pro Lys Lys Leu Pro Pro Arg Pro
                90                  95                  100

ggc ggc cct gaa ccc aag cca gga cac cct ccc caa aca agg cag gct 990

Gly Gly Pro Glu Pro Lys Pro Gly His Pro Pro Gln Thr Arg Gln Ala
            105                 110                 115

aca gcc cgg act gtg acc cca aaa gga cag ctt ccc gga ggc aag gca 1038

Thr Ala Arg Thr Val Thr Pro Lys Gly Gln Leu Pro Gly Gly Lys Ala
        120                 125                 130

ccc cca aaa gca gga tct gtc ccc agc tcc ttc ctg ctg aag aag gcc 1086

Pro Pro Lys Ala Gly Ser Val Pro Ser Ser Phe Leu Leu Lys Lys Ala
    135                 140                 145

agg gag ccc ggg ccc cca cga gag ccc aag gag ccg ttt cgc cca ccc 1134

Arg Glu Pro Gly Pro Pro Arg Glu Pro Lys Glu Pro Phe Arg Pro Pro
150                 155                 160                 165

ccc atc aca ccc cac gag tac atg ctc tcg ctg tac agg acg ctg tcc 1182

Pro Ile Thr Pro His Glu Tyr Met Leu Ser Leu Tyr Arg Thr Leu Ser
                170                 175                 180

gat gct gac aga aag gga ggc aac agc agc gtg aag ttg gag gct ggc     1230

Asp Ala Asp Arg Lys Gly Gly Asn Ser Ser Val Lys Leu Glu Ala Gly
            185                 190                 195

ctg gcc aac acc atc acc agc ttt att gac aaa ggg caa gat gac cga     1278

Leu Ala Asn Thr Ile Thr Ser Phe Ile Asp Lys Gly Gln Asp Asp Arg
        200                 205                 210

ggt ccc gtg gtc agg aag cag agg tac gtg ttt gac att agt gcc ctg     1326

Gly Pro Val Val Arg Lys Gln Arg Tyr Val Phe Asp Ile Ser Ala Leu
    215                 220                 225

gag aag gat ggg ctg ctg ggg gcc gag ctg cgg atc ttg cgg aag aag     1374

Glu Lys Asp Gly Leu Leu Gly Ala Glu Leu Arg Ile Leu Arg Lys Lys
230                 235                 240                 245

ccc tcg gac acg gcc aag cca gcg gcc ccc gga ggc ggg cgg gct gcc     1422

Pro Ser Asp Thr Ala Lys Pro Ala Ala Pro Gly Gly Gly Arg Ala Ala
                250                 255                 260

cag ctg aag ctg tcc agc tgc ccc agc ggc cgg cag ccg gcc tcc ttg     1470

Gln Leu Lys Leu Ser Ser Cys Pro Ser Gly Arg Gln Pro Ala Ser Leu
            265                 270                 275

ctg gat gtg cgc tcc gtg cca ggc ctg gac gga tct ggc tgg gag gtg     1518

Leu Asp Val Arg Ser Val Pro Gly Leu Asp Gly Ser Gly Trp Glu Val
        280                 285                 290

ttc gac atc tgg aag ctc ttc cga aac ttt aag aac tcg gcc cag ctg     1566

Phe Asp Ile Trp Lys Leu Phe Arg Asn Phe Lys Asn Ser Ala Gln Leu
    295                 300                 305

tgc ctg gag ctg gag gcc tgg gaa cgg ggc agg gcc gtg gac ctc cgt     1614

Cys Leu Glu Leu Glu Ala Trp Glu Arg Gly Arg Ala Val Asp Leu Arg
310                 315                 320                 325

ggc ctg ggc ttc gac cgc gcc gcc cgg cag gtc cac gag aag gcc ctg     1662

Gly Leu Gly Phe Asp Arg Ala Ala Arg Gln Val His Glu Lys Ala Leu
                330                 335                 340

ttc ctg gtg ttt ggc cgc acc aag aaa cgg gac ctg ttc ttt aat gag     1710

Phe Leu Val Phe Gly Arg Thr Lys Lys Arg Asp Leu Phe Phe Asn Glu
            345                 350                 355

att aag gcc cgc tct ggc cag gac gat aag acc gtg tat gag tac ctg 1758

Ile Lys Ala Arg Ser Gly Gln Asp Asp Lys Thr Val Tyr Glu Tyr Leu
        360                 365                 370

ttc agc cag cgg cga aaa cgg cgg gcc cca ctg gcc act cgc cag ggc 1806

Phe Ser Gln Arg Arg Lys Arg Arg Ala Pro Leu Ala Thr Arg Gln Gly
    375                 380                 385

aag cga ccc agc aag aac ctt aag gct cgc tgc agt cgg aag gca ctg 1854

Lys Arg Pro Ser Lys Asn Leu Lys Ala Arg Cys Ser Arg Lys Ala Leu
390                 395                 400                 405

cat gtc aac ttc aag gac atg ggc tgg gac gac tgg atc atc gca ccc 1902

His Val Asn Phe Lys Asp Met Gly Trp Asp Asp Trp Ile Ile Ala Pro
                410                 415                 420

ctt gag tac gag gct ttc cac tgc gag ggg ctg tgc gag ttc cca ttg 1950

Leu Glu Tyr Glu Ala Phe His Cys Glu Gly Leu Cys Glu Phe Pro Leu
            425                 430                 435

cgc tcc cac ctg gag ccc acg aat cat gca gtc atc cag acc ctg atg 1998

Arg Ser His Leu Glu Pro Thr Asn His Ala Val Ile Gln Thr Leu Met
        440                 445                 450

aac tcc atg gac ccc gag tcc aca cca ccc acc tgc tgt gtg ccc acg 2046

Asn Ser Met Asp Pro Glu Ser Thr Pro Pro Thr Cys Cys Val Pro Thr
    455                 460                 465

cgg ctg agt ccc atc agc atc ctc ttc att gac tct gcc aac aac gtg 2094

Arg Leu Ser Pro Ile Ser Ile Leu Phe Ile Asp Ser Ala Asn Asn Val
470                 475                 480                 485

gtg tat aag cag tat gag gac atg gtc gtg gag tcg tgt ggc tgc agg 2142

Val Tyr Lys Gln Tyr Glu Asp Met Val Val Glu Ser Cys Gly Cys Arg
                490                 495                 500

tagcagcact ggccctctgt cttcctgggt ggcacatccc aagagcccct tcctgcactc 2202

ctggaatcac agaggggtca ggaagctgtg gcaggagcat ctacacagct tgggtgaaag 2262

gggattccaa taagcttgct cgctctctga gtgtgacttg ggctaaaggc ccccttttat 2322

ccacaagttc ccctggctga ggattgctgc ccgtctgctg atgtgaccag tggcaggcac 2382

aggtccaggg agacagactc tgaatgggac tgagtcccag gaaacagtgc tttccgatga 2442

gactcagccc accatttctc ctcacctggg ccttctcagc ctctggactc tcctaagcac 2502

ctctcaggag agccacaggt gccactgcct cctcaaatca catttgtgcc tggtgacttc 2562

ctgtccctgg gacagttgag aagctgactg ggcaagagtg ggagagaaga ggagagggct 2622

tggatagagt tgaggagtgt gaggctgtta gactgttaga tttaaatgta tattgatgag 2682

ataaaaagca aaactgtgcc t 2703

<210> 2
<211> 501
<212> PRT
<213> Homo sapiens

<400> 2
Met Arg Leu Pro Lys Leu Leu Thr Phe Leu Leu Trp Tyr Leu Ala Trp
1               5                   10                  15

Leu Asp Leu Glu Phe Ile Cys Thr Val Leu Gly Ala Pro Asp Leu Gly
            20                  25                  30

Gln Arg Pro Gln Gly Thr Arg Pro Gly Leu Ala Lys Ala Glu Ala Lys
        35                  40                  45

Glu Arg Pro Pro Leu Ala Arg Asn Val Phe Arg Pro Gly Gly His Ser
    50                  55                  60

Tyr Gly Gly Gly Ala Thr Asn Ala Asn Ala Arg Ala Lys Gly Gly Thr
65                  70                  75                  80

Gly Gln Thr Gly Gly Leu Thr Gln Pro Lys Lys Asp Glu Pro Lys Lys
                85                  90                  95

Leu Pro Pro Arg Pro Gly Gly Pro Glu Pro Lys Pro Gly His Pro Pro
            100                 105                 110

Gln Thr Arg Gln Ala Thr Ala Arg Thr Val Thr Pro Lys Gly Gln Leu
        115                 120                 125

Pro Gly Gly Lys Ala Pro Pro Lys Ala Gly Ser Val Pro Ser Ser Phe
    130                 135                 140

Leu Leu Lys Lys Ala Arg Glu Pro Gly Pro Pro Arg Glu Pro Lys Glu
145                 150                 155                 160

Pro Phe Arg Pro Pro Pro Ile Thr Pro His Glu Tyr Met Leu Ser Leu
                165                 170                 175

Tyr Arg Thr Leu Ser Asp Ala Asp Arg Lys Gly Gly Asn Ser Ser Val
            180                 185                 190

Lys Leu Glu Ala Gly Leu Ala Asn Thr Ile Thr Ser Phe Ile Asp Lys
        195                 200                 205

Gly Gln Asp Asp Arg Gly Pro Val Val Arg Lys Gln Arg Tyr Val Phe
    210                 215                 220

Asp Ile Ser Ala Leu Glu Lys Asp Gly Leu Leu Gly Ala Glu Leu Arg
225                 230                 235                 240

Ile Leu Arg Lys Lys Pro Ser Asp Thr Ala Lys Pro Ala Ala Pro Gly
                245                 250                 255

Gly Gly Arg Ala Ala Gln Leu Lys Leu Ser Ser Cys Pro Ser Gly Arg
            260                 265                 270

Gln Pro Ala Ser Leu Leu Asp Val Arg Ser Val Pro Gly Leu Asp Gly
        275                 280                 285

Ser Gly Trp Glu Val Phe Asp Ile Trp Lys Leu Phe Arg Asn Phe Lys
    290                 295                 300

Asn Ser Ala Gln Leu Cys Leu Glu Leu Glu Ala Trp Glu Arg Gly Arg
305                 310                 315                 320

Ala Val Asp Leu Arg Gly Leu Gly Phe Asp Arg Ala Ala Arg Gln Val
                325                 330                 335

His Glu Lys Ala Leu Phe Leu Val Phe Gly Arg Thr Lys Lys Arg Asp
            340                 345                 350

Leu Phe Phe Asn Glu Ile Lys Ala Arg Ser Gly Gln Asp Asp Lys Thr
        355                 360                 365

Val Tyr Glu Tyr Leu Phe Ser Gln Arg Arg Lys Arg Arg Ala Pro Leu
    370                 375                 380

Ala Thr Arg Gln Gly Lys Arg Pro Ser Lys Asn Leu Lys Ala Arg Cys
385                 390                 395                 400

Ser Arg Lys Ala Leu His Val Asn Phe Lys Asp Met Gly Trp Asp Asp
                405                 410                 415

Trp Ile Ile Ala Pro Leu Glu Tyr Glu Ala Phe His Cys Glu Gly Leu
            420                 425                 430

Cys Glu Phe Pro Leu Arg Ser His Leu Glu Pro Thr Asn His Ala Val
        435                 440                 445

Ile Gln Thr Leu Met Asn Ser Met Asp Pro Glu Ser Thr Pro Pro Thr
    450                 455                 460

Cys Cys Val Pro Thr Arg Leu Ser Pro Ile Ser Ile Leu Phe Ile Asp
465                 470                 475                 480

Ser Ala Asn Asn Val Val Tyr Lys Gln Tyr Glu Asp Met Val Val Glu
                485                 490                 495

Ser Cys Gly Cys Arg
            500

<210> 3
<211> 2703
<212> DNA
<213> Homo sapiens

<220>
<221> CDS
<222> (640)..(2142)

<220>
<221> misc_feature
<222> (2032)..(2034)
<223> codon coding for any amino acid except cysteine

<400> 1
ccatggcctc gaaagggcag cggtgatttt tttcacataa atatatcgca cttaaatgag 60

tttagacagc atgacatcag agagtaatta aattggtttg ggttggaatt ccgtttccaa 120

ttcctgagtt caggtttgta aaagattttt ctgagcacct gcaggcctgt gagtgtgtgt 180

gtgtgtgtgt gtgtgtgtgt gtgtgtgtga agtattttca ctggaaagga ttcaaaacta 240

gggggaaaaa aaaactggag cacacaggca gcattacgcc attcttcctt cttggaaaaa 300

tccctcagcc ttatacaagc ctccttcaag ccctcagtca gttgtgcagg agaaaggggg 360

cggttggctt tctcctttca agaacgagtt attttcagct gctgactgga gacggtgcac 420

gtctggatac gagagcattt ccactatggg actggataca aacacacacc cggcagactt 480

caagagtctc agactgagga gaaagccttt ccttctgctg ctactgctgc tgccgctgct 540

tttgaaagtc cactcctttc atggtttttc ctgccaaacc agaggcacct ttgctgctgc 600

cgctgttctc tttggtgtca ttcagcggct ggccagagg atg aga ctc ccc aaa 654
Met Arg Leu Pro Lys
1               5

ctc ctc act ttc ttg ctt tgg tac ctg gct tgg ctg gac ctg gaa ttc 702
Leu Leu Thr Phe Leu Leu Trp Tyr Leu Ala Trp Leu Asp Leu Glu Phe
                10                  15                  20

atc tgc act gtg ttg ggt gcc cct gac ttg ggc cag aga ccc cag ggg 750
Ile Cys Thr Val Leu Gly Ala Pro Asp Leu Gly Gln Arg Pro Gln Gly
            25                  30                  35

acc agg cca gga ttg gcc aaa gca gag gcc aag gag agg ccc ccc ctg 798
Thr Arg Pro Gly Leu Ala Lys Ala Glu Ala Lys Glu Arg Pro Pro Leu
        40                  45                  50

gcc cgg aac gtc ttc agg cca ggg ggt cac agc tat ggt ggg ggg gcc 846
Ala Arg Asn Val Phe Arg Pro Gly Gly His Ser Tyr Gly Gly Gly Ala
    55                  60                  65

acc aat gcc aat gcc agg gca aag gga ggc acc ggg cag aca gga ggc 894
Thr Asn Ala Asn Ala Arg Ala Lys Gly Gly Thr Gly Gln Thr Gly Gly
70                  75                  80                  85

ctg aca cag ccc aag aag gat gaa ccc aaa aag ctg ccc ccc aga ccg 942
Leu Thr Gln Pro Lys Lys Asp Glu Pro Lys Lys Leu Pro Pro Arg Pro
                90                  95                  100

ggc ggc cct gaa ccc aag cca gga cac cct ccc caa aca agg cag gct 990
Gly Gly Pro Glu Pro Lys Pro Gly His Pro Pro Gln Thr Arg Gln Ala
            105                 110                 115

aca gcc cgg act gtg acc cca aaa gga cag ctt ccc gga ggc aag gca 1038
Thr Ala Arg Thr Val Thr Pro Lys Gly Gln Leu Pro Gly Gly Lys Ala
        120                 125                 130

ccc cca aaa gca gga tct gtc ccc agc tcc ttc ctg ctg aag aag gcc 1086
Pro Pro Lys Ala Gly Ser Val Pro Ser Ser Phe Leu Leu Lys Lys Ala
    135                 140                 145

agg gag ccc ggg ccc cca cga gag ccc aag gag ccg ttt cgc cca ccc 1134

Arg Glu Pro Gly Pro Pro Arg Glu Pro Lys Glu Pro Phe Arg Pro Pro
150                 155                 160                 165

ccc atc aca ccc cac gag tac atg ctc tcg ctg tac agg acg ctg tcc     1182

Pro Ile Thr Pro His Glu Tyr Met Leu Ser Leu Tyr Arg Thr Leu Ser
                170                 175                 180

gat gct gac aga aag gga ggc aac agc agc gtg aag ttg gag gct ggc     1230

Asp Ala Asp Arg Lys Gly Gly Asn Ser Ser Val Lys Leu Glu Ala Gly
            185                 190                 195

ctg gcc aac acc atc acc agc ttt att gac aaa ggg caa gat gac cga     1278

Leu Ala Asn Thr Ile Thr Ser Phe Ile Asp Lys Gly Gln Asp Asp Arg
        200                 205                 210

ggt ccc gtg gtc agg aag cag agg tac gtg ttt gac att agt gcc ctg     1326

Gly Pro Val Val Arg Lys Gln Arg Tyr Val Phe Asp Ile Ser Ala Leu
    215                 220                 225

gag aag gat ggg ctg ctg ggg gcc gag ctg cgg atc ttg cgg aag aag     1374

Glu Lys Asp Gly Leu Leu Gly Ala Glu Leu Arg Ile Leu Arg Lys Lys
230                 235                 240                 245

ccc tcg gac acg gcc aag cca gcg gcc ccc gga ggc ggg cgg gct gcc     1422

Pro Ser Asp Thr Ala Lys Pro Ala Ala Pro Gly Gly Gly Arg Ala Ala
                250                 255                 260

cag ctg aag ctg tcc agc tgc ccc agc ggc cgg cag ccg gcc tcc ttg     1470

Gln Leu Lys Leu Ser Ser Cys Pro Ser Gly Arg Gln Pro Ala Ser Leu
            265                 270                 275

ctg gat gtg cgc tcc gtg cca ggc ctg gac gga tct ggc tgg gag gtg     1518

Leu Asp Val Arg Ser Val Pro Gly Leu Asp Gly Ser Gly Trp Glu Val
        280                 285                 290

ttc gac atc tgg aag ctc ttc cga aac ttt aag aac tcg gcc cag ctg     1566

Phe Asp Ile Trp Lys Leu Phe Arg Asn Phe Lys Asn Ser Ala Gln Leu
    295                 300                 305

tgc ctg gag ctg gag gcc tgg gaa cgg ggc agg gcc gtg gac ctc cgt     1614

Cys Leu Glu Leu Glu Ala Trp Glu Arg Gly Arg Ala Val Asp Leu Arg
310                 315                 320                 325

ggc ctg ggc ttc gac cgc gcc gcc cgg cag gtc cac gag aag gcc ctg     1662

Gly Leu Gly Phe Asp Arg Ala Ala Arg Gln Val His Glu Lys Ala Leu

                330                 335                 340

ttc ctg gtg ttt ggc cgc acc aag aaa cgg gac ctg ttc ttt aat gag 1710

Phe Leu Val Phe Gly Arg Thr Lys Lys Arg Asp Leu Phe Phe Asn Glu
            345                 350                 355

att aag gcc cgc tct ggc cag gac gat aag acc gtg tat gag tac ctg 1758

Ile Lys Ala Arg Ser Gly Gln Asp Asp Lys Thr Val Tyr Glu Tyr Leu
        360                 365                 370

ttc agc cag cgg cga aaa cgg cgg gcc cca ctg gcc act cgc cag ggc 1806

Phe Ser Gln Arg Arg Lys Arg Arg Ala Pro Leu Ala Thr Arg Gln Gly
    375                 380                 385

aag cga ccc agc aag aac ctt aag gct cgc tgc agt cgg aag gca ctg 1854

Lys Arg Pro Ser Lys Asn Leu Lys Ala Arg Cys Ser Arg Lys Ala Leu
390                 395                 400                 405

cat gtc aac ttc aag gac atg ggc tgg gac gac tgg atc atc gca ccc 1902

His Val Asn Phe Lys Asp Met Gly Trp Asp Asp Trp Ile Ile Ala Pro
                410                 415                 420

ctt gag tac gag gct ttc cac tgc gag ggg ctg tgc gag ttc cca ttg 1950

Leu Glu Tyr Glu Ala Phe His Cys Glu Gly Leu Cys Glu Phe Pro Leu
            425                 430                 435

cgc tcc cac ctg gag ccc acg aat cat gca gtc atc cag acc ctg atg 1998

Arg Ser His Leu Glu Pro Thr Asn His Ala Val Ile Gln Thr Leu Met
        440                 445                 450

aac tcc atg gac ccc gag tcc aca cca ccc acc nnn tgt gtg ccc acg 2046

Asn Ser Met Asp Pro Glu Ser Thr Pro Pro Thr Xaa Cys Val Pro Thr
    455                 460                 465

cgg ctg agt ccc atc agc atc ctc ttc att gac tct gcc aac aac gtg 2094

Arg Leu Ser Pro Ile Ser Ile Leu Phe Ile Asp Ser Ala Asn Asn Val
470                 475                 480                 485

gtg tat aag cag tat gag gac atg gtc gtg gag tcg tgt ggc tgc agg 2142

Val Tyr Lys Gln Tyr Glu Asp Met Val Val Glu Ser Cys Gly Cys Arg
                490                 495                 500

tagcagcact ggccctctgt cttcctgggt ggcacatccc aagagcccct tcctgcactc 2202

ctggaatcac agaggggtca ggaagctgtg gcaggagcat ctacacagct tgggtgaaag 2262

gggattccaa taagcttgct cgctctctga gtgtgacttg ggctaaaggc cccctttat 2322

ccacaagttc ccctggctga ggattgctgc ccgtctgctg atgtgaccag tggcaggcac 2382

aggtccaggg agacagactc tgaatgggac tgagtcccag gaaacagtgc tttccgatga 2442

gactcagccc accatttctc ctcacctggg ccttctcagc ctctggactc tcctaagcac 2502

ctctcaggag agccacaggt gccactgcct cctcaaatca catttgtgcc tggtgacttc 2562

ctgtccctgg gacagttgag aagctgactg ggcaagagtg ggagagaaga ggagagggct 2622

tggatagagt tgaggagtgt gaggctgtta gactgttaga tttaaatgta tattgatgag 2682

ataaaaagca aaactgtgcc t 2703

<210> 4
<211> 501
<212> PRT
<213> Homo sapiens

<220>
<221> misc_feature
<222> (465)
<223> any amino acid except cysteine

<400> 2
Met Arg Leu Pro Lys Leu Leu Thr Phe Leu Leu Trp Tyr Leu Ala Trp
1               5                   10                  15

Leu Asp Leu Glu Phe Ile Cys Thr Val Leu Gly Ala Pro Asp Leu Gly
            20                  25                  30

Gln Arg Pro Gln Gly Thr Arg Pro Gly Leu Ala Lys Ala Glu Ala Lys
        35                  40                  45

Glu Arg Pro Pro Leu Ala Arg Asn Val Phe Arg Pro Gly Gly His Ser
    50                  55                  60

Tyr Gly Gly Gly Ala Thr Asn Ala Asn Ala Arg Ala Lys Gly Gly Thr
65                  70                  75                  80

Gly Gln Thr Gly Gly Leu Thr Gln Pro Lys Lys Asp Glu Pro Lys Lys
                85                  90                  95

Leu Pro Pro Arg Pro Gly Gly Pro Glu Pro Lys Pro Gly His Pro Pro
            100                 105                 110

Gln Thr Arg Gln Ala Thr Ala Arg Thr Val Thr Pro Lys Gly Gln Leu
        115                 120                 125

Pro Gly Gly Lys Ala Pro Pro Lys Ala Gly Ser Val Pro Ser Ser Phe
    130                 135                 140

Leu Leu Lys Lys Ala Arg Glu Pro Gly Pro Pro Arg Glu Pro Lys Glu
145                 150                 155                 160

Pro Phe Arg Pro Pro Pro Ile Thr Pro His Glu Tyr Met Leu Ser Leu
                165                 170                 175

Tyr Arg Thr Leu Ser Asp Ala Asp Arg Lys Gly Gly Asn Ser Ser Val
            180                 185                 190

Lys Leu Glu Ala Gly Leu Ala Asn Thr Ile Thr Ser Phe Ile Asp Lys
        195                 200                 205

Gly Gln Asp Asp Arg Gly Pro Val Val Arg Lys Gln Arg Tyr Val Phe
    210                 215                 220

Asp Ile Ser Ala Leu Glu Lys Asp Gly Leu Leu Gly Ala Glu Leu Arg
225                 230                 235                 240

Ile Leu Arg Lys Lys Pro Ser Asp Thr Ala Lys Pro Ala Ala Pro Gly
                245                 250                 255

Gly Gly Arg Ala Ala Gln Leu Lys Leu Ser Ser Cys Pro Ser Gly Arg
            260                 265                 270

Gln Pro Ala Ser Leu Leu Asp Val Arg Ser Val Pro Gly Leu Asp Gly
        275                 280                 285

Ser Gly Trp Glu Val Phe Asp Ile Trp Lys Leu Phe Arg Asn Phe Lys
    290                 295                 300

Asn Ser Ala Gln Leu Cys Leu Glu Leu Glu Ala Trp Glu Arg Gly Arg
305                 310                 315                 320

Ala Val Asp Leu Arg Gly Leu Gly Phe Asp Arg Ala Ala Arg Gln Val
                325                 330                 335

His Glu Lys Ala Leu Phe Leu Val Phe Gly Arg Thr Lys Lys Arg Asp
            340                 345                 350

Leu Phe Phe Asn Glu Ile Lys Ala Arg Ser Gly Gln Asp Asp Lys Thr
        355                 360                 365

Val Tyr Glu Tyr Leu Phe Ser Gln Arg Arg Lys Arg Arg Ala Pro Leu
    370                 375                 380

Ala Thr Arg Gln Gly Lys Arg Pro Ser Lys Asn Leu Lys Ala Arg Cys
385                 390                 395                 400

Ser Arg Lys Ala Leu His Val Asn Phe Lys Asp Met Gly Trp Asp Asp
                405                 410                 415

Trp Ile Ile Ala Pro Leu Glu Tyr Glu Ala Phe His Cys Glu Gly Leu
            420                 425                 430

Cys Glu Phe Pro Leu Arg Ser His Leu Glu Pro Thr Asn His Ala Val
        435                 440                 445

Ile Gln Thr Leu Met Asn Ser Met Asp Pro Glu Ser Thr Pro Pro Thr
    450                 455                 460

Xaa Cys Val Pro Thr Arg Leu Ser Pro Ile Ser Ile Leu Phe Ile Asp
465                 470                 475                 480

Ser Ala Asn Asn Val Val Tyr Lys Gln Tyr Glu Asp Met Val Val Glu
                485                 490                 495

Ser Cys Gly Cys Arg
            500